## Europäisches Patentamt

(19) **European Patent Office**

**Office européen des brevets**

(11) Veröffentlichungsnummer: **0 167 085**
**B1**

# (12) EUROPÄISCHE PATENTSCHRIFT

(45) Veröffentlichungstag der Patentschrift:
04.10.89

(51) Int. Cl.⁴: **C 11 D 17/00, C 11 D 3/22**

(21) Anmeldenummer: **85107819.6**

(22) Anmeldetag: **24.06.85**

(54) **Mittel zur Toilettenreinigung.**

(30) Priorität: **02.07.84 DE 3424317**

(43) Veröffentlichungstag der Anmeldung:
**08.01.86 Patentblatt 86/2**

(45) Bekanntmachung des Hinweises auf die Patenterteilung:
**04.10.89 Patentblatt 89/40**

(84) Benannte Vertragsstaaten:
**AT BE CH DE FR GB IT LI NL**

(56) Entgegenhaltungen:
**EP-A-0 101 402**
**WO-A-82/01319**
**AU-B-494 380**
**FR-A-2 227 321**
**GB-A-2 021 143**
**US-A-2 560 097**
**US-A-3 248 333**

(73) Patentinhaber: **Henkel Kommanditgesellschaft auf Aktien, Postfach 1100 Henkelstrasse 67, D-4000 Düsseldorf- Holthausen (DE)**

(72) Erfinder: **Menke, Ronald, Nietzschestrasse 9, D-4020 Mettmann 2 (DE)**
Erfinder: **Holdt, Bernd, Gutenbergstrasse 45, D-4000 Düsseldorf (DE)**
Erfinder: **Praus, Gerd, Höppnerstrasse 80, D-4150 Krefeld (DE)**
Erfinder: **Plantikow, Petra, Virchowstrasse 29, D-4030 Ratingen 8 (DE)**

**Beschreibung**

Blockförmige Mittel, die Reinigungswirkstoffe enthalten, sind für verschiedene Zwecke bekannt. So werden beispielsweise in der US-2 560 097 leicht lösliche Handwaschtabletten für den einmaligen Gebrauch beschrieben, die neben ionischen Tensiden Sägespäne als Füllstoff enthalten. In der AU-B 494 380 werden schnell zerfallende Desodorierungstabletten beschrieben, die neben Parfüm und Dispergiermittel mikrokristalline Cellulose als Füllstoff enthalten.

Zur Reinhaltung von Toilettenschüsseln sind im Laufe der Zeit eine Reihe selbsttätiger chemischer Verfahren entwickelt worden. So kennt man aus der DOS-2 910 955 beispielsweise ein Verfahren, bei dem zu Blöcken geformte Wirkstoffe in den Wasservorratsbehälter der Toilette eingelegt werden und sich dort allmählich auflösen, so daß das in die Spülschüssel eintretende Wasser über längere Zeit einen zur Reinigung ausreichenden Gehalt an Wirkstoffen aufweist. Dieses Verfahren ist naturgemäß nur bei solchen Toiletten anwendbar, die mit Wasservorratsbehältern betrieben werden und bei denen diese Behälter frei zugänglich sind.

Eine andere verbreitete Methode zur Reinhaltung von Toilettenschüsseln besteht darin, einen Vorrat eines Reinigungsmittels in der Toilettenschüssel selbst, zweckmäßigerweise unter dem Innenrand, in einer solchen Weise anzubringen, daß bei jedem Spülvorgang ein kleiner Teil des Mittels vom zulaufenden Spülwasser gelöst und in der Schüssel verbreitet wird. Zur richtigen Dosierung der festen oder flüssigen Mittel sind komplizierte Spendervorrichtungen vorgeschlagen worden, die aber einen aufwendigen Herstellungsgang erfordern und meist nicht universell anwendbar sind. Weite Verbreitung haben dagegen einfache Systeme gefunden, bei denen das Reinigungsmittel als Block in einem käfigartigen Behälter vorliegt, der bei jedem Spülvorgang vom Wasser durchströmt wird. Hier bestehen die Schwierigkeiten darin, solche Wirkstoffkombinationen zu finden, die einerseits zu formbeständigen, nicht zerfließenden Blöcken geformt werden können, andererseits so ausreichende Löslichkeit besitzen, daß während der kurzen Spülphase genügend Reinigungsmittel an das Wasser abgegeben wird.

Insbesondere dann, wenn mehrere Wirkstoffe mit unterschiedlichen Aufgaben, wie Tenside, Komplexbildner, Desinfektionsmittel oder Parfüm, aus den Blöcken heraus abgegeben werden sollen, stellen diese einfachen Systeme nur Kompromißlösungen dar. Es ist in der Regel nicht möglich, alle Wirkstoffe gleichzeitig in solchen Konzentrationen in die Blöcke einzuarbeiten, daß die abgespülten Mengen optimal für die angestrebten Wirkungen wären, ohne daß die Blöcke die gewünschte Festigkeit verlören.

Es bestand daher die Aufgabe, die Mittel des Standes der Technik zu verbessern und weiterzuentwickeln.

Gegenstand der vorliegenden Erfindung sind Toilettenreinigungsmittel in stückiger Form zum Einsatz in Toilettenspülbecken, die neben Tensiden und anderen üblichen Bestandteilen solcher Mittel weitgehend von Lignin und anderen Verunreinigungen befreites Cellulosepulver mit einer Teilchengröße zwischen 5 und 800 µm enthalten.

Die erfindungsgemäßen Mittel weisen eine hohe Formbeständigkeit auf und zeigen ein ausgezeichnetes Verhalten bei der Freisetzung der Wirkstoffe. Ganz besonders bemerkenswert ist die außerordentlich geringe Neigung zum Zerfließen im Kontakt mit Wasser, die es ermöglicht, mehrere Stücke des Mittels im selben Behälter anzuwenden, ohne daß diese miteinander verkleben. In einer besonderen Ausführungsform liegen die Mittel der Erfindung deshalb in Form mehrerer kleinerer Stücke, d. h. in Form eines Granulates vor.

Vorzugsweise enthalten die Mittel
a) anionische Tenside
b) nichtionische Tenside
c) Parfüm und
d) Cellulosepulver

Weiterhin können
e) Abspülregulatoren
f) anorganische Salze
g) Komplexbildner
h) Kalkstein lösende Säuren
i) antimikrobielle Wirkstoffe
k) Plastifikatoren und
l) andere übliche Zusätze enthalten sein.

Bei dem enthaltenen Cellulosepulver (d) handelt es sich um weitgehend von Lignin und anderen Verunreinigungen befreite, insbesondere gebleichte Cellulosen. Die Art der chemischen Vorbehandlung ist insoweit unerheblich, wie sichergestellt ist, daß die gereinigten Cellulosen noch die hier wesentlichen Eigenschaften der nativen Cellulose aufweisen. Das heißt, sie sollen noch die faserartige Struktur des nativen Materials, die aus partiell kristallinen Fibrillen aufgebaut ist, besitzen und sie dürfen in Wasser nicht löslich sein und nicht bis zur Gelbildung quellen. Insbesondere werden chemisch nicht modifizierte Cellulosen eingesetzt.

Die Teilchengröße des Cellulosepulvers wird in erster Linie durch die Forderung bestimmt, daß in der Toilettenschüssel keine auffälligen Rückstände aus der allmählichen Auflösung der Reinigungsmittelstücke verbleiben dürfen. Daher liegt die Obergrenze für die Teilchengröße im allgemeinen bei 800 µm; vorzugsweise werden aber Pulver verwendet, deren Teilchen kleiner als 300 µm sind, wobei Teilchengrößen bis herunter zu 5 µm üblich sind. Wegen produktionstechnischer Vorteile werden die Pulver häufig in einer lose agglomerierten oder granulierten Form eingesetzt.

Das Cellulosepulver ist in den erfindungsgemäßen Mitteln in Mengen von vorzugsweise wenigstens 2 Gewichtsprozent vorhanden; besonders bevorzugt sind Mengen von 5 Gewichtsprozent und mehr. Die Obergrenze des Cellulosegehaltes wird durch die an sich unerwünschte Verdünnung der Wirkstoffe in den Rezepturen bei steigendem Cellulosegehalt bestimmt, so daß die Mittel in der Regel nicht mehr als 30 Gewichtsprozent, vorzugsweise nicht mehr als 15 Gewichtsprozent an Cellulosepulver aufweisen.

Als anionische Tenside (a) finden in den erfindungsgemäßen Reinigungsmitteln insbesondere solche von Sulfat- und Sulfonattyp Verwendung, doch können auch andere Typen, wie Seifen oder Salze von Fettsäurecyanamiden eingesetzt werden. Die anionischen Tenside werden vorwiegend in Form ihrer Natriumsalze verwendet.

Besonders geeignete Tenside vom Sulfattyp sind die Schwefelsäuremonoester von primären Alkoholen natürlichen und synthetischen Ursprungs, d. h. von Fettalkoholen, wie z. B. Kokosfettalkoholen, Talgfettalkoholen, Oleylalkohol, oder $C_{10}$-$C_{20}$-Oxoalkoholen und solche von sekundären Alkoholen dieser Kettenlängen. Daneben kommen die Schwefelsäuremonoester der mit 1 - 6 Mol Ethylenoxid ethoxylierten aliphatischen primären Alkohole bzw. ethoxylierten sekundären Alkohole bzw. Alkylphenole in Betracht.

Bei den Tensiden vom Sulfonattyp handelt es sich in erster Linie um die Alkylbenzolsulfonate mit $C_{9-15}$-Alkylgruppen und um die Olefinsulfonate; das sind Gemische aus Alken- und Hydroxyalkansulfonaten sowie Disulfonaten, wie man sie beispielsweise aus Monoolefinen mit end- oder innenständiger Doppelbindung durch Sulfonieren mit gasförmigem Schwefeltrioxid und anschließender alkalischer oder saurer Hydrolyse der Sulfonierungsprodukte erhält. Weitere brauchbare Tenside vom Sulfonattyp sind die Alkansulfonate, die aus $C_{12}$ - $C_{18}$-Alkanen durch Sulfochlorierung oder Sulfoxidation und anschließende Hydrolyse bzw. Neutralisation oder durch Bisulfitaddition an Olefine erhältlich sind, sowie die Ester von α -Sulfofettsäuren, z. B. die α -sulfonierten Methyl- oder Ethylester der hydrierten Kokos-, Palmkern- oder Talgfettsäuren.

Als nichtionische Tenside (b) eignen sich für die erfindungsgemäßen Mittel insbesondere Anlagerungsprodukte von 1 - 100, vorzugsweise 5 - 60 Mol Ethylenoxid an 1 Mol einer aliphatischen oder alkylaromatischen Verbindung mit im wesentlichen 10 - 20 Kohlenstofatomen aus der Gruppe der Alkohole, Alkylphenole, Fettsäuren und Fettsäureamide, doch lassen sich auch andere Typen, wie die Ethylenoxidaddukte an Polypropylenoxid und die Kondensationsprodukte aus langkettigen primären Alkoholen und Glucose, verwenden. Besonders geeignet sind die Anlagerungsprodukte von 20 - 50 Mol Ethylenoxid an primäre Alkohole, wie zum Beispiel Kokos- oder Talgfettalkohole, an Oleylalkohol, an Oxoalkohole der entsprechenden Kettenlängen, oder an entsprechende sekundäre Alkohole, sowie an Mono-oder Dialkylphenole mit 6 - 14 C-Atomen in den Alkylresten.

Die Auswahl der Tenside und ihr Anteil in den erfindungsgemäßen Mitteln wird von der beabsichtigten Reinigungsleistung, aber auch vom Zusammenwirken mit den anderen Bestandteilen nicht nur bei der Anwendung, sondern auch bei der Herstellung und Lagerung der Mittel bestimmt. Von den oben genannten anionischen Tensiden werden deshalb Alkylbenzolsulfonate, Olefinsulfonate und Fettalkoholsulfate besonders bevorzugt. Der Anteil der anionischen Tenside am gesamten Mittel liegt im allgemeinen zwischen 2 und 40 Gewichtsprozent; mit Anteilen zwischen 5 und 25 Gewichtsprozent werden regelmäßig sehr gute Reinigungsleistungen erzielt.

Besonders bevorzugte nichtionische Tenside sind die Anlagerungsprodukte von Ethylenoxid an langkettige aliphatische Alkohole mit 12 - 18 C-Atomen und an Alkylphenole mit 6 - 12 C-Atomen in der Alkylgruppe, insbesondere solche Produkte, deren durchschnittlicher Gehalt an Ethylenoxid zwischen 20 und 50 Mol pro Mol Tensid beträgt. Der Anteil der nichtionischen Tenside in den erfindungsgemäßen Mitteln liegt im allgemeinen zwischen 5 und 60 Gewichtsprozent, vorzugsweise aber zwischen 7 und 50 Gewichtsprozent.

Aufgabe der Komponente c, des Parfüms, ist es in erster Linie, während der Anwendung der Mittel der Umgebungsluft einen angenehmen Duft zu vermitteln. Insbesondere dann, wenn Parfümöle, d. h. flüssige Produkte eingesetzt werden, tragen diese aber auch zur Plastifizierung der Rohmasse bei und bestimmen wesentlich die Konsistenz der fertigen Reinigungsmittelstücke. Besonders bemerkenswert ist, daß in Gegenwart des Cellulosepulvers sehr große Anteile an Parfümölen eingearbeitet werden können, ohne daß zu weiche und deshalb wenig brauchbare Stücke entstehen. Derartig hohe Anteile sind vor allem dann erwünscht, wenn die Mittel einen sehr intensiven Duft über längere Zeit abgeben sollen. Der Gehalt an Parfüm kann in den erfindungsgemäßen Mitteln zwischen etwa 1 und etwa 25 Gewichtsprozent betragen, vorzugsweise liegt er zwischen 4 und 15 Gewichtsprozent. Bei der Auswahl der Parfüms, die in der Regel Gemische mehrerer verschiedener Riechstoffe sind, ist, mehr noch als bei den anderen Bestandteilen der Mittel, darauf zu achten, daß Unverträglichkeiten mit anderen Bestandteilen beispielsweise Desinfektionsmitteln oder Säuren, vermieden werden.

Neben den Bestandteilen a - d können die erfindungsgemäßen Mittel weitere Bestandteile e - l enthalten. Es sind dies im einzelnen:

e) Abspülregulatoren.

Die so bezeichneten Substanzen dienen einerseits dazu, den Verbrauch der Mittel während des Einsatzes so zu steuern, daß die vorgesehene Standzeit eingehalten wird, andererseits sollen sie dazu beitragen, die abgespülten Mengen pro Spülvorgang möglichst konstant zu halten. Die Wirkung dieser Regulatoren wird durch den Einsatz des Cellulosepulvers in unerwarteter Weise verbessert und ergänzt, so daß in vielen Fällen ihr Anteil an den Mitteln gegenüber herkömmlichen Formulierungen stark reduziert werden kann. Als Regulatoren eignen sich vorzugsweise feste langkettige Fettsäuren, wie Stearinsäure, Fettsäureethanolamide, wie Kokosfettsäuremonoethanolamid, oder feste Polyethylenglykole, wie solche mit Molekulargewichten zwischen 10 000 und 50 000. Im allgemeinen werden nicht mehr als 25 Gewichtsprozent an Abspülregulatoren in den Mitteln benötigt. Der bevorzugte Bereich liegt bei 5 bis 15 Gewichtsprozent.

## f) Anorganische Salze

Durch den Zusatz anorganischer Salze lassen sich ebenfalls die Konsistenz und das Abspülverhalten der Reinigungsmittelstücke beeinflussen, doch verbessert diese Komponente auch die Homogenität der Stücke in kritischen Fällen. Die Salze können weiterhin durch Elektrolyteffekte und als Puffer die Reinigungswirkung der Tenside verstärken und sie können, beispielsweise die Polyphosphate, als Härte bindende Stoffe wirken.

Als Salze werden im allgemeinen die Alkali- oder Ammoniumsalze von Mineralsäuren gegebenenfalls in hydratisierter Form verwendet. Bevorzugt werden die Natriumsalze der Schwefelsäure, der Phosphorsäuren, der Kohlensäure und der Salzsäure. Sie werden in Mengen bis zu 50 Gewichtsprozent, vorzugsweise in Mengen von 10 bis 40 Gewichtsprozent eingesetzt.

## g) Komplexbildner

Als Wirkstoffe gegen die Wasserhärte, insbesondere aber gegen die in Wässern häufig anzutreffenden Schwermetallionen, wie die des Eisens und des Mangans, können die erfindungsgemäßen Mittel Komplexbildner enthalten. Geeignet sind solche Verbindungen, die gegenüber diesen Schwermetallionen eine so starke komplexierende Wirkung entfalten, daß eine Ausfällung der Metallhydroxide bei Anwendungskonzentration nicht stattfinden kann, beispielsweise Ethylendiamintetraessigsäure, 1-Hydroxyethan- 1,1-diphosphonsäure oder Polyacrylsäure und deren Salze. Die einzusetzenden Mengen richten sich nach dem Komplexiervermögen der Komplexbildner und der vorgesehenen Abspülgeschwindigkeit. Im allgemeinen enthalten die Mittel deshalb nicht mehr als 30 Gewichtsprozent Komplexbildner, meist werden schon mit Mengen bis zu 15 Gewichtsprozent befriedigende Ergebnisse erzielt.

## h) Kalkstein lösende Säuren

Zur Verhinderung von Kalksteinablagerungen und zur Entfernung alter Beläge in den Toilettenschüsseln können den Mitteln als Wirkstoffe Säuren zugesetzt werden. Geeignet sind wasserlösliche, nichtflüchtige, insbesondere feste Säuren, die einen $pk_s$ -Wert über $10^{-4}$, vorzugsweise über $10^{-2}$ aufweisen und keine schwerlöslichen Calciumsalze bilden, beispielsweise Amidosulfonsäure, Phosphorsäure und Zitronensäure. Ihr Anteil an den Reinigungsmitteln kann bis zu 30 Gewichtsprozent, vorzugsweise 5 bis 20 Gewichtsprozent betragen.

## i) Antimikrobielle Wirkstoffe

Häufig ist es erwünscht, mit der Toilettenreinhaltung auch eine keimhemmende Behandlung zu verbinden. Zu diesem Zweck enthalten die erfindungsgemäßen Mittel bis zu 10 Gewichtsprozent, vorzugsweise bis zu 5 Gewichtsprozent antimikrobielle Wirkstoffe. Diese Wirkstoffe können den verschiedensten chemischen Klassen entstammen, doch ist bei der Auswahl auch hier auf die Verträglichkeit mit den anderen Komponenten der Mittel zu achten. Geeignete Wirkstoffe sind beispielsweise Phenole, Aktivchlor abspaltende Mittel, oder die unter der Bezeichnung Kathon® gehandelten Mikrobistatika mit Isothiazolonstruktur.

## k) Plastifikatoren

Die Plastifikatoren dienen in erster Linie dazu, der Reinigungsmittelmasse eine für die Formung der Stücke geeignete Plastizität zu verleihen. Es handelt sich dabei um hochsiedende organische Substanzen, die bei der Verarbeitungstemperatur flüssig sind. Als Plastifikatoren eignen sich beispielsweise Paraffinöle, 1,2-Propylenglykol, Silikonöle, Phthalsäureester, Terpene und Dihydroabietinsäuremethylester. Vorzugsweise werden Dihydroabietinsäuremethylester, Diethylphthalat und Dibutylphthalat eingesetzt. Da die Funktion der Plastifikatoren in vielen Fällen von anderen Bestandteilen der Mittel, vor allem von den Parfümölen aber auch von nichtionischen Tensiden oder flüssigen Säuren übernommen werden kann, ist ihre Anwesenheit nur in wenigen Fällen notwendig. Sie werden dann in Mengen bis zu 15 Gewichtsprozent eingesetzt.

## l) Andere übliche Zusätze

Neben den bisher genannten Komponenten können die erfindungsgemäßen Mittel noch andere übliche Zusätze enthalten, die das Eigenschaftsbild der Reinigungsmittel abrunden. Als Beispiele für derartige Zusätze sind Farbstoffe und Konservierungsmittel besonders hervorzuheben; ihr Anteil an den Mitteln kann bis zu 5 Gewichtsprozent betragen.

Neben der Möglichkeit, auch hoch parfümhaltige Mischungen zu formstabilen Stücken zu verarbeiten, und dem ausgezeichneten Abspülverhalten besitzen die erfindungsgemäßen Mittel noch weitere Vorteile. Durch die Einarbeitung des Cellulosepulvers erreicht man überraschenderweise auch eine stärkere Duftabgabe an die Umgebungsluft. Dies ist vor allem dann von Bedeutung, wenn die Rezepturgestaltung aus anderen Gründen die Einarbeitung hoher Parfümmengen nicht zuläßt. Durch die schnellere Abgabe der Duftstoffe vermitteln nun auch solche, an Parfüm ärmere Mischungen einen so überzeugenden Geruchseindruck, wie er vom Verbraucher gewünscht wird.

Gegenstand der Erfindung ist ebenfalls ein Verfahren zur Reinhaltung von Toiletten unter Anwendung der erfindungsgemäßen Mittel. Es ist dadurch gekennzeichnet, daß man die Mittel an geeigneten Haltern oder in korb- oder käfigartigen Behältern im Toilettenbecken an einer Stelle anbringt, die bei jedem Spülvorgang vom zulaufenden Spülwasser durchströmt wird. Eine wenig auffällige und störende Anbringung wird erreicht, wenn die Mittel unter den Innenrand des Toilettenbeckens eingehängt werden.

Die Herstellung der erfindungsgemäßen Reinigungsmittelstücke und Reinigungsmittelgranulate kann auf verschiedenen Wegen erfolgen. So lassen sich beispielsweise Stücke durch Eingießen von entsprechenden flüssigen Vorgemischen in Formen, wo sie erstarren, oder durch Verpressen von mehr oder weniger trockenen Vorgemischen zu Einzelstücken herstellen. Granulate lassen sich beispielsweise durch Agglomerationsgranulierung herstellen. Bevorzugt ist jedoch für beide Formen das Strangpreßverfahren, bei dem ein plastifizierbares Vorgemisch mit Hilfe eines Extruders zu einem endlosen Strang geformt und dieser in Stücke geschnitten wird. Auf diese Weise können Reinigungsmittelstücke in Form von Säulen mit beliebigen Querschnitten und Höhen hergestellt werden. Das Strangpreßverfahren ist daher ebenfalls ein Gegenstand der Erfindung. Abhängig vom Abspülverhalten und der erwünschten Standzeit besitzen die für den Einzeleinsatz bestimmten Stücke im allgemeinen Anfangsgewichte von 10 bis 100, vorzugsweise 20 bis 50 g.

Ein besonders bemerkenswerter Vorteil der cellulosehaltigen Reinigungsmittelstücke besteht in ihrer geringen Neigung zur Klebrigkeit und zum Zerfließen im Kontakt mit Wasser. Dadurch wird die Anwendungssicherheit der Stücke bei herkömmlichem Gebrauch, d. h. beim Einsatz einzelner Blöcke oder Kegel, ganz wesentlich verbessert. Die Neigung zur Klebrigkeit ist aber so gering, daß es mit den neuen Mitteln gelingt, auch mehrere Stücke in ein und demselben Behälter unterzubringen, ohne daß diese nach dem Kontakt mit Wasser miteinander verschmelzen. Diese Aufteilung der Gesamtreinigungsmittelmenge auf mehrere kleinere Einzelstücke ist sehr erwünscht, weil so die Oberfläche im Verhältnis zum Inhalt der Mittel vergrößert und damit die Abspülgeschwindigkeit erhöht werden kann. Auf diese Weise ist es jetzt möglich, auch bei schwer abspülbaren Mischungen innerhalb der kurzen Kontaktzeiten während des Spülvorganges stets eine ausreichende Wirkstoffabgabe an das Wasser zu gewährleisten. Auch die Parfümabgabe an die Umgebungsluft wird durch die Vergrößerung der Oberfläche weiter erleichtert. Deshalb besteht ein bevorzugtes Verfahren zur Reinhaltung von Toiletten unter Anwendung der erfindungsgemäßen Mittel darin, mehrere unabhängige Reinigungsmittelstücke, insbesondere mehr als zehn, zusammen in einem Behälter anzuwenden. Stücke, die für dieses Verfahren vorgesehen sind, haben kleinere Abmessungen als solche, die einzeln verwendet werden. Sie wiegen im allgemeinen zwischen 0,05 und 10 Gramm, vorzugsweise zwischen 0, 1 und 2 Gramm. Vorzugsweise werden Stücke mit überwiegend nicht ebenen Oberflächen verwendet, um sicherzustellen, daß sich die Stücke in geschütteter Form möglichst nicht flächig berühren können und die hohe Gesamtoberfläche weitgehend erhalten bleibt. Durch Strangpressen hergestellte Stücke bzw. Einzelteile von Granulaten besitzen daher vorzugsweise kreisförmigen oder ovalen Querschnitt und werden vorzugsweise auf solche Längen geschnitten, daß das Verhältnis von Länge zu größtem Durchmesser über 1, insbesondere zwischen 1 und 2 liegt.

Bei der Kombination aus mehreren Reinigungsmittelstücken und einem Behälter ergibt sich als weiterer Vorteil die Möglichkeit, Reinigungsmittelstücke unterschiedlicher Zusammensetzung gemeinsam einzusetzen. Dies ist dann von besonderer Bedeutung, wenn zwei oder mehr Wirkstoffe, die wegen ihrer chemischen Unverträglichkeit nicht ohne weiteres in einem Stück kombiniert werden können, gleichzeitig angewendet werden sollen. Beispiele solcher Wirkstoffe sind starke Säuren oder Hypochlorit einerseits und säure- oder oxidationsempfindliche Farb- oder Duftstoffe andererseits, für deren gemeinsame Anwendung bis in jüngste Zeit nach einem unkomplizierten Verfahren gesucht wurde. Der Einsatz mehrerer unabhängiger Reinigungsmittelstücke unterschiedlicher Zusammensetzung in einem Behälter stellt daher eine weitere hervorzuhebende Ausführungsform des erfindungsgemäßen Verfahrens dar.

**Beispiele**

Sofern nicht anders angegeben, wurden die anionischen Tenside, die Komplexbildner und die anorganischen Salze in den angeführten Kompositionen in Form der Natriumsalze eingesetzt. Folgende Abkürzungen werden in den Beispielen verwendet:

EDTA - Ethylendiamintetraessigsäuretetranatriumsalz
EO - Ethylenoxid (in nichtionischen Tensiden)
Kathon 886 MW) Antimikrobielle Wirkstoffe mit
Iso- Kathon 893) thiazolonstruktur
PE-Glykol 10 000 - Polyethylenglykol mit Molekulargewicht 10 000

1. Reinigungsmittelwürfel mit hohem Parfümgehalt
In einem Trommelmischer wurde in Form eines losen Granulates ein Vorgemisch der folgenden Zusammensetzung hergestellt:

Laurylsulfat                                    15, 0 Gew.-%

| | |
|---|---|
| Talgalkohol + 50 EO | 22, 0 Gew.-% |
| EDTA | 9, 5 Gew.-% |
| Stearinsäure | 3, 0 Gew.-% |
| Gebleichte, pulverisierte | |
| Cellulose (Korngröße bis ca. 50 µm) | 30, 0 Gew.-% |
| Citrusterpen | 20, 0 Gew.-% |
| Konservierungsmittel | 0, 5 Gew.-% |

Es ließ sich mit Hilfe eines Extruders kontinuierlich zu einem Strang von quadratischem Querschnitt mit 2 cm Kantenlänge verformen, der direkt nach dem Verlassen der Düse von einem rotierenden Messer in Würfel geschnitten wurde. Die trotz des hohen Parfümgehaltes festen, formstabilen Würfel klebten nicht und konnten in geschütteter Form, ohne miteinander zu verbacken, gelagert werden.

Beispiele 2 bis 6

In ähnlicher Weise wie im Beispiel 1 wurden Vorgemische der im folgenden angegebenen Zusammensetzungen hergestellt und durch Strangpressen zu Reinigungsmittelstücken weiterverarbeitet:

Alkalisches Reinigungsmittel

| | |
|---|---|
| Fettalkoholsulfat $C_{12}/C_{14}$ | 15 Gew.-% |
| Talgalkohol + 50 EO | 25 Gew.-% |
| Parfüm | 12 Gew.-% |
| Gebleichte Cellulose | |
| (Korngröße bis ca. 150 µm) | 20 Gew.-% |
| Stearinsäure | 3 Gew.-% |
| PE-Glykol 20 000 | 10 Gew.-% |
| $Na_2SO_4$ | 8 Gew.-% |
| $Na_2CO_3$ | 6 Gew.-% |
| Konservierungsmittel | 1 Gew.-% |

Aus dieser Masse wurden zylindrische Stücke von 4 mm Durchmesser und 10 mm Länge geformt, die für die weitere Anwendung in Mengen von je ca. 80 Stück in käfigartige Behälter verpackt wurden. Die Behälter selbst waren aus elastischem Kunststoff in Form eines länglichen Korbes gefertigt, dessen angespritzer Deckel zum Nachfüllen des Mittels angehoben werden konnte.

Antimikrobiell wirkendes Reinigungsmittel

| | |
|---|---|
| Olefinsulfonat $C_{15}$-$C_{18}$ | 10 Gew.-% |
| Talgalkohol + 50 EO | 30 Gew.-% |
| Parfüm | 6 Gew.-% |
| Cellulosepulver | |
| (Korngröße bis ca. 90 µm) | 15 Gew.-% |
| Stearinsäure | 3 Gew.-% |
| PE-Glykol 10 000 | 10 Gew.-% |
| $Na_2SO_4$ | 17,5 Gew.-% |
| $Na_2CO_3$ | 4 Gew.-% |
| Kathon 886 MW | 3 Gew.-% |
| Kathon 893 | 1,5 Gew.-% |

Aus dieser Mischung wurden für den Einsatz als Einzelstücke sechskantige Säulen von 4,2 cm$^2$ Querschnitt und 6 cm Länge gefertigt.

Neutrales Reinigungsmittel zur Verhinderung von Ablagerungen

| | |
|---|---|
| Kokosalkoholsulfat | 15 Gew.-% |
| Kokosalkohol + 35 EO | 28 Gew.-% |
| Parfüm | 6 Gew.-% |
| Cellulosepulver | |
| (Korngröße bis ca. 750 µm) | 10 Gew.-% |
| Stearinsäure | 3 Gew.-% |
| PE-Glykol 20 000 | 10 Gew.-% |
| $Na_2SO_4$ | 17 Gew.-% |
| EDTA | 10 Gew.-% |
| Konservierungsmittel | 1 Gew.-% |

Die vorliegende Mischung wurde zu einem Reinigungsmittelgranulat verarbeitet, das zur Anwendung in hartem, eisenhaltigen Wasser bestimmt ist. Die Einzelstücke hatten zylindrische Form mit 6 mm Durchmesser und 7 mm Höhe.

Mittel zur Auflösung von Kalkablagerungen

6

| Dodecylbenzolsulfonat | 20 Gew.-% |
|---|---|
| Talalkohol + 40 EO | 28 Gew.-% |
| Säurestabiles Parfüm (Acidofix Apfelbouquet) | 4 Gew.-% |
| Cellulosepulver (Korngröße bis ca. 100 μm) | 8 Gew.-% |
| Stearinsäure | 6 Gew.-% |
| Kokosfettsäuremonoethanolamid | 7 Gew.-% |
| $Na_2SO_4$ | 12 Gew.-% |
| Amidosulfonsäure | 15 Gew.-% |

Durch Strangpressen wurde die Mischung in die Form von kurzen Zylindern mit einem Durchmesser von 1,8 cm und einer Länge von 2,5 cm gebracht, die einzeln, bei Bedarf aber auch zu mehreren Stücken gleichzeitig in einem Behälter eingesetzt werden können. Trotz des niedrigen Parfümgehaltes verströmte das Produkt einen kräftigen Duft.

### Reinigungsblock

| Alkylbenzolsulfonat | 25,0 Gew.-% |
|---|---|
| Talgalkohol + 50 EO | 7,5 Gew.-% |
| Parfüm | 6,0 Gew.-% |
| Granulierte α-Cellulose (Teilchengröße des Pulvers bis 125 μm) | 15,0 Gew.-% |
| Hydrierte Talgfettsäure | 1,5 Gew.-% |
| $Na_2SO_4$ | 37,5 Gew.-% |
| $Na_2CO_3$ | 2,0 Gew.-% |
| EDTA | 4,5 Gew.-% |
| Konservierungsmittel | 1,0 Gew.-% |

Durch Extrudieren wurden Flachstränge mit 1,4 und 6 cm Kantenlänge erzeugt, die dann in Stücke von ca. 35 g geschnitten wurden. Die Haltbarkeit dieser Stücke betrug 300 - 400 Spülungen, wenn sie in Plastikkörbchen unter dem Innenrand der Toilettenschüssel placiert wurden.

### Demonstration der verstärkten Parfümabgabe

Zur Demonstration der verstärkten Parfümabgabe aus den Cellulosepulver enthaltenden Reinigungsmittelstücken wurden zwei bis auf den Cellulosegehalt nahezu gleiche Mittel über einen längeren Zeitraum gelagert, wobei in bestimmten Abständen der Parfümverlust gravimetrisch bestimmt wurde. Dazu waren je 35 g der beiden Mittel in Form kleiner Zylinder von 5 mm Durchmesser und 7 mm Höhe ungeordnet in Körbchen geschüttet worden, die dann offen in Klimaräumen aufbewahrt wurden. Die beiden Mittel hatten folgende Zusammensetzung (in Gewichtsprozenten):

| Wirkstoffe | Formulierung I (erfindungsgemäß) | Formulierung II (Vergleich) |
|---|---|---|
| Dodecylbenzolsulfonat | 5,0 | 5,0 |
| Talgalkohol + 50 EO | 39,0 | 44,0 |
| Parfüm Geißblatt | 6,0 | 6,0 |
| Cellulosepulver (Korngröße bis ca. 200 μm) | 10,0 | - |
| Stearinsäure | 3,0 | 3,0 |
| PE-Glykol 20 000 | 10,0 | 10,0 |
| $Na_2SO_4$ | 17,0 | 22,0 |
| EDTA | 9,0 | 9,0 |
| Konservierungsmittel | 1,0 | 1,0 |

Wurden die beiden Mittel bei 30°C und 25 % relativer Luftfeuchtigkeit gelagert, so ergaben sich folgende Gewichtsabnahmen in Gramm:

| Lagerzeit/Tage | I | II |
|---|---|---|
| 1 | 0,184 | 0,014 |
| 3 | 0,491 | 0,135 |
| 7 | 0,561 | 0,337 |

7

| | | |
|---|---|---|
| 9 | 0,633 | 0,418 |
| 11 | 0,753 | 0,482 |
| 14 | 0,831 | 0,592 |
| 18 | 0,953 | 0,695 |

Aus den Meßwerten ist deutlich der positve Einfluß zu erkennen, den das Cellulosepulver auf die Geschwindigkeit der Parfümabgabe ausübt.

Granulat aus Stücken unterschiedlicher Zusammensetzung

Granulat-Rezeptur A (in Gewichtsprozent) mit säureempfindlichem Duftstoff, rosa eingefärbt

| | |
|---|---|
| Dodecylbenzolsulfonat | 15,0 |
| Talgfettalkohol 50 EO | 33,0 |
| Stearinsäure | 4,0 |
| Cellulosepulver (Korngröße bis etwa 100 µm) | 15,0 |
| säureempfindlicher Duftstoff | 8,0 |
| Hyazinthe 0/219880 (Dragoco) | |
| EDTA | 5,0 |
| Natriumsulfat | 20,0 |
| | 100,0 |

| | |
|---|---|
| Diazzofarbstoff C. J. 26050 | 0,005 |

Granulat-Rezeptur B (in Gewichtsprozent) mit Säurekomponente zur Auflösung von Kalkablagerungen

| | |
|---|---|
| Dodecylbenzolsulfonat | 18,0 |
| Talgfettalkohol 50 EO | 34,0 |
| Stearinsäure | 4,0 |
| Kokosfettsäuremonoethanolamid | 10,0 |
| Cellulosepulver (Korngröße bis etwa 100 µm) | 10,0 |
| Dihydroabietinsäuremethylester | 4,0 |
| Amidosulfonsäure | 20,0 |
| | 100,0 |

Die pulverförmigen Rohstoffe der Rezepturen A und B wurden je 10 Minuten in Lödige-Mischern von je 300 kg Füllvolumen vorgemischt und anschließend kontinuierlich über Banddosierwaagen zwei Extrudieranlagen zugeführt. Parfüm bzw. Plastifikator wurden direkt in die Extruder dosiert.

Die aus einer Lochscheibe austretenden Stränge mit kreisförmigem Querschnitt und einem Durchmesser von 4 mm wurden durch rotierende Messer auf Längen von etwa 7 mm geschnitten und die anfallenden Stücke in Form zweier rieselfähiger Granulate aufgefangen. Beide Granulate wurden in einer Mischtrommel gemischt und über eine Dosieranlage in Portionen von 50 g in die für die Anwendung geeigneten käfigartigen Kunststoffbehälter gefüllt.

Bestimmung der Abspülgeschwindigkeit

Die mit Granulat gefüllten Behälter aus Beispiel 8 wurden unter dem Innenrand einer DIN-Tiefspültoilette der Firma Keramag eingehängt, die über eine Steueranlage in Abständen von 1 Stunde mit je 9 l Wasser gespült wurde. Nach 300 Spülgängen waren die Einzelstücke des Granulates noch nicht miteinander verschmolzen, obwohl die Masse der Teilchen stark abgenommen hatte. Diese Testdauer entspricht einer Praxisanwendung über mehrere Wochen. Erst nach 360 Spülungen waren die Wirkstoffe verbraucht.

**Patentansprüche**

1. Toilettenreinigungsmittel in stückiger Form, enthaltend Tenside und andere übliche Bestandteile solcher Mittel, dadurch gekennzeichnet, daß es weitgehend von Lignin und anderen Verunreinigungen befreites Cellulosepulver mit einer Teilchengröße zwischen 5 und 800 µm enthält.

2. Toilettenreinigungsmittel nach Anspruch 1, enthaltend
 a) anionisches Tensid,
 b) nichtionisches Tensid,
 c) Parfüm und
 d) Cellulosepulver.

3. Toilettenreinigungsmittel nach einem der Ansprüche 1 oder 2, dadurch gekennzeichnet, daß es e) einen Abspülregulator enthält.

4. Toilettenreinigungsmittel nach einem der Ansprüche 1 bis 3, dadurch gekennzeichnet, daß es f) anorganisches Salz enthält.

5. Toilettenreinigungsmittel nach einem der Ansprüche 1 bis 4, dadurch gekennzeichnet, daß es g) einen Komplexbildner oder h) Kalkstein lösende Säure enthält.

6. Toilettenreinigungsmittel nach einem der Ansprüche 1 bis 5, dadurch gekennzeichnet, daß es einen antimikrobiellen Wirkstoff enthält.

7. Toilettenreinigungsmittel nach einem der Ansprüche 1 bis 6, bestehend aus
a) 2 bis 40 Gew.-% anionischem Tensid
b) 5 bis 60 Gew.-% nichtionischem Tensid
c) 1 bis 25 Gew.-% Parfüm
d) 2 bis 30 Gew.-% Cellulosepulver
e) 0 bis 25 Gew.-% Abspülregulator
f) 0 bis 50 Gew.-% anorganischem Salz
g) 0 bis 30 Gew.-% Komplexbildner
h) 0 bis 30 gew.-5% Kalkstein lösender Säure
i) 0 bis 10 Gew.-% antimikrobiellem Wirkstoff
k) 0 bis 15 Gew.-% Plastifikator
l) 0 bis 5 Gew.-% weiteren Zusätzen.

8. Toilettenreinigungsmittel nach einem der Ansprüche 1 bis 7, bestehend aus
a) 5 - 25 Gew.-% anionischem Tensid aus der Gruppe Alkylbenzolsulfonat, Alkylsulfat und Olefinsulfonat oder deren Mischungen
b) 7 - 50 Gew.-% nichtionischem Tensid aus der Gruppe der Anlagerungsprodukte von 20 - 50 Mol Ethylenoxid an langkettige aliphatische Alkohole mit 12 - 18 C-Atomen oder an Alkylphenole mit 6 - 12 C-Atomen in der Alkylgruppe oder deren Mischungen
c) 4 - 15 Gew.-% Parfüm
d) 5 - 15 Gew.-% Cellulosepulver mit einer Teilchengröße zwischen 5 und 800 µm
e) 5 - 15 Gew.-% Abspülregulator aus der Gruppe langkettige Fettsäuren, Fettsäureethanolamide und feste Polyethylenglykole mit Molekulargewichten zwischen 10 000 und 50 000 oder deren Mischungen
f) 10 - 40 Gew.-% anorganischem Salz aus der Gruppe der Natriumsalze der Schwefelsäure, der Phosphorsäuren, der Kohlensäure oder der Salzsäure oder deren Mischungen
g) 0 - 15 Gew.-% Komplexbildner
h) 0 - 30 Gew.-% Kalkstein lösende Säure
i) 0 - 5 Gew.-% antimikrobiellem Wirkstoff
k) 0 - 15 Gew.-% Plastifikator
l) 0 - 5 Gew.-% anderen üblichen Zusätzen.

9. Toilettenreinigungsmittel nach einem der Ansprüche 1 bis 8, dadurch gekennzeichnet, daß das Cellulosepulver eine Teilchengröße zwischen 5 und 300 µm aufweist.

10. Toilettenreinigungsmittel nach einem der Ansprüche 1 bis 9, dadurch gekennzeichnet, daß es in Form eines Granulates vorliegt.

11. Toilettenreinigungsmittel nach Anspruch 10, dadurch gekennzeichnet, daß die Einzelstücke 0,05 bis 10 Gramm wiegen und die Form einer Säule mit kreisförmigem oder ovalem Querschnitt aufweisen, bei der das Verhältnis von Länge zu größtem Durchmesser zwischen 1 und 2 liegt.

12. Verfahren zur Herstellung von Toilettenreinigungsmitteln, bei dem eine Grundmasse durch einen Extruder zu einem Strang geformt wird und dieser in Stücke geschnitten wird, dadurch gekennzeichnet, daß die Grundmasse eine der in den Ansprüchen 1 bis 11 angegebenen Zusammensetzungen hat.

13. Verfahren zur Reinhaltung von Toiletten, bei dem ein Toilettenreinigungsmittel an einem Halter oder in einem Korb- oder käfigartigen Behälter im Toilettenbecken an einer Stelle angebracht wird, die bei jedem Spülvorgang vom zulaufenden Spülwasser durchströmt wird, dadurch gekennzeichnet, daß ein Toilettenreinigungsmittel nach einem der Ansprüche 1 bis 11 verwendet wird.

14. Verfahren nach Anspruch 13, bei dem sich mehrere Reinigungsmittelstücke in demselben Behälter befinden.

15. Verfahren nach Anspruch 14, bei dem die Stücke unterschiedliche Zusammensetzung aufweisen.


## Claims

1. A piece-form toilet cleaner containing surfactants and other constituents typical of toilet cleaners, characterized in that it contains cellulose powder substantially freed from lignin and other impurities and having a particle size of from 5 to 800 µm.

2. A toilet cleaner as claimed in claim 1 containing
a) anionic surfactant,
b) nonionic surfactant,
c) perfume and
d) cellulose powder.

3. A toilet cleaner as claimed in claim 1 or 2, characterized in that it contains e) an erosion regulator.

4. A toilet cleaner as claimed in any of claims 1 to 3, characterized in that it contains f) inorganic salt.

5. A toilet cleaner as claimed in any of claims 1 to 4, characterized in that it contains g) a complexing agent or h) limestone-dissolving acid.

6. A toilet cleaner as claimed in any of claims 1 to 5, characterized in that it contains an antimicrobial agent.

7. A toilet cleaner as claimed in any of claims 1 to 6 consisting of

a) 2 to 40 % by weight anionic surfactant
b) 5 to 60 % by weight nonionic surfactant
c) 1 to 25 % by weight perfume
d) 2 to 30 % by weight cellulose powder
e) 0 to 25 % by weight erosion regulator
f) 0 to 50 % by weight inorganic salt
g) 0 to 30 % by weight complexing agent
h) 0 to 30 % by weight limestone-dissolving acid
i) 0 to 10 % by weight antimicrobial agent
k) 0 to 15 % by weight plasticizer
l) 0 to 5 % by weight other additives.

8. A toilet cleaner as claimed in any of claims 1 to 7 consisting of

a) 5 to 25 % by weight anionic surfactant from the group consisting of alkyl benzene sulfonate, alkyl sulfate and olefin sulfonate or mixtures thereof

b) 7 to 50 % by weight nonionic surfactant from the group consisting of adducts of 20 to 50 mol ethylene oxide with long-chain aliphatic alcohols containing 12 to 18 carbon atoms or with alkyl phenols containing 6 to 12 carbon atoms in the alkyl group or mixtures thereof

c) 4 to 15 % by weight perfume

d) 5 to 15 % by weight cellulose powder having a particle size of 5 to 800 μm

e) 5 to 15 % by weight erosion regulator from the group consisting of long-chain fatty acids, fatty acid ethanolamides and solid polyethylene glycols having molecular weight of from 10, 000 to 50, 000 or mixtures thereof

f) 10 to 40 % by weight inorganic salt from the group consisting of the sodium salts of sulfuric acid, phosphorus acids, carbonic acid or hydrochloric acid or mixtures thereof

g) 0 to 15 % by weight complexing agent
h) 0 to 30 % by weight limestone-dissolving acid
i) 0 to 5 % by weight antimicrobial agent
k) 0 to 15 % by weight plasticizer
l) 0 to 5 % by weight other standard additives.

9. A toilet cleaner as claimed in any of claims 1 to 8, characterized in that the cellulose powder has a particle size of from 5 to 300 μm.

10. A toilet cleaner as claimed in any of claims 1 to 9, characterized in that it is present in the form of a granulate.

11. A toilet cleaner as claimed in claim 10, characterized in that the individual pieces weigh 0. 05 to 10 grams and are in the form of a column of circular or oval cross-section in which the ratio of length to largest diameter is between 1 and 2.

12. A process for the production of toilet cleaners in which a basic mass is extruded to a strand which is then cut into pieces, characterized in that the basic mass has one of the compositions claimed in claims 1 to 11.

13. A process for keeping toilets clean, in which a toilet cleaner is arranged on a holder or in a basket or cage-like container in the toilet bowl at a place where the inflowing flushing water flows past whenever the toilet is flushed, characterized in that the toilet cleaner claimed in any of claims 1 to 11 is used.

14. A process as claimed in claim 13, in which several pieces of the cleaner are present in the same container.

15. A process as claimed in claim 14 in which the pieces are of different composition.

**Revendications**

1. Nettoyant pour toilettes en morceaux, renfermant des tensio-actifs et d'autres ingrédients usuels, caractérisé en ce qu'il renferme de la poudre de cellulose en grande partie débarrassée de lignine et d'autres impuretés, dont la taille des particules est comprise entre 5 et 800 μm.

2. Nettoyant pour toilettes selon la revendication 1, renfermant les ingrédients suivants:

a) tensio-actif anionique
b) tensio-actif non ionique
c) parfum et
d) poudre de cellulose.

3. Nettoyant pour toilettes selon l'une des revendications 1 ou 2, caractérisé en ce qu'il renferme e) un régulateur de rinçage.

4. Nettoyant pour toilettes selon l'une des revendications 1 à 3, caractérisé en ce qu'il renferme f) un sel inorganique.

5. Nettoyant pour toilettes selon l'une des revendications 1 à 4, caractérisé en ce qu'il renferme g) un agent complexant ou h) un acide dissolvant le tartre.

6. Nettoyant pour toilettes selon l'une des revendications 1 à 5, caractérisé en ce qu'il renferme un agent antimicrobien.

7. Nettoyant pour toilettes selon l'une des revendications 1 à 6, constitué de

a) 2 à 40 % en poids de tensio-actif anionique

b) 5 à 60 % en poids de tensio-actif non ionique

c) 1 à 25 % en poids de parfum

d) 2 à 30 % en poids de de poudre de cellulose

e) 0 à 25 % en poids de régulateur de rinçage

f) 0 à 50 % en poids de sel inorganique

g) 0 à 30 % en poids d'agent complexant

h) 0 à 30 % en poids d'acide dissolvant le tartre

i) 0 à 10 % en poids d'agent antimicrobien

k) 0 à 15 % en poids de plastifiant

l) 0 à 5 % en poids d'autres addititfs.

8. Nettoyant pour toilettes selon l'une des revendications 1 à 7, constitué de

a) 5 à 25 % en poids de tensio-actif anionique du groupe des alkylbenzènsulfonates, des alkylsulfates et des sulfates oléfiniques ou des mélanges de ceux-ci

b) 7 à 50 % en poids de tensio-actif non ionique du groupe des produits d'addition de 20 à 50 moles d'oxyde d'éthylène à des alcools aliphatiques à chaîne longue comportant 12 à 18 atomes de carbone ou à des alkylphénols comportant 6 à 12 atomes de carbone dans le groupement alkyle, ou des mélanges de ceux-ci

c) 4 à 15 % en poids de parfum

d) 5 à 15 % en poids de poudre de cellulose, dont la taille des particules est comprise entre 5 et 800 µm

e) 5 à 15 % en poids de régulateur de rinçage du groupe acides gras à longue chaîne, éthanolamide d'acide gras et polyéthylèneglycols solides de poids moléculaire compris entre 10 000 et 50 000, ou des mélanges de ceux-ci

f) 10 à 40 % en poids de sel inorganique du groupe des sels de sodium de l'acide sulfurique, des acides phosphoriques, de l'acide carbonique ou de l'acide chlorhydrique, ou des mélanges de ceux-ci

g) 0 à 15 % en poids d'agents complexants

h) 0 à 30 % en poids d'acide dissolvant le tartre

i) 0 à 5 % en poids d'agent antimicrobien

k) 0 à 15 % en poids de plastifiant

l) 0 à 15 % en poids d'autres additifs usuels.

9. Nettoyant pour toilettes selon l'une des revendications 1 à 8, caractérisé en ce que la poudre de cellulose présente une taille de particules comprise entre 5 et 300 µm

10. Nettoyant pour toilettes selon l'une des revendications 1 à 9, caractérisé en ce qu'il se présente sous la forme d'un granulat.

11. Nettoyant pour toilettes selon la revendication 10, caractérisé en ce que les morceaux séparés pèsent 0, 05 à 10 g et sont en forme de colonne à section circulaire ou ovale, dont le rapport entre la longueur et le plus grand diamètre est compris entre 1 et 2.

12. Procédé de fabrication de nettoyants pour toilettes, dans lequel une pâte est formée à l'aide d'une extrudeuse en un boudin, qui est découpé ensuite en morceaux, caractérisé en ce que la dite pâte possède une des compositions spécifiées aux revendications 1 à 11.

13. Procédé de maintien de la propreté des toilettes dans lequel un nettoyant pour toilettes sur un support ou dans un boîtier de type panier ou cage, est disposé dans la cuvette des toilettes à un endroit qui est inondé par l'eau de rinçage à chaque actionnement de la chasse, caractérisé en ce qu'un nettoyant pour toilettes selon l'une des revendications 1 à 11 est utilisé.

14. Procédé selon la revendication 13, dans lequel plusieurs morceaux de nettoyant se trouvent dans le même boîtier.

15. Procédé selon la revendication 14, dans lequel les morceaux présentent une composition différente.